(19) Europäisches Patentamt
European Patent Office
Office européen des brevets

(11) EP 1 530 964 A1

(12) **DEMANDE DE BREVET EUROPEEN**

(43) Date de publication:
**18.05.2005 Bulletin 2005/20**

(51) Int Cl.[7]: **A61K 7/42**

(21) Numéro de dépôt: **04292444.9**

(22) Date de dépôt: **14.10.2004**

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(30) Priorité: **17.11.2003 FR 0350843**

(71) Demandeur: **L'OREAL**
**75008 Paris (FR)**

(72) Inventeurs:
- **Vernaire, Sandrine**
  **92310 Sèvres (FR)**
- **Josso, Martin**
  **75007 Paris (FR)**

(74) Mandataire: **Miszputen, Laurent**
**L'OREAL - D.I.P.I.**
**25-29 Quai Aulagnier**
**92600 Asnières (FR)**

(54) **Composition solide photoprotectrice à base d'un sel d'amidon esterifié par l'anhydride octenylsuccinique ; utilisations**

(57) La présente demande concerne une composition solide photoprotectrice comprenant dans un milieu physiologiquement acceptable

a) au moins une phase grasse,
b) au moins un filtre organique UV,
c) au moins des nanopigments minéraux à base d'oxydes métalliques,
d) au moins un sel d'amidon estérifié par l'anhydride octénylsuccinique.

La présente demande concerne également l'utilisation d'au moins un sel d'amidon estérifié par l'anhydride octénylsuccinique dans une composition solide photoprotectrice comprenant au moins une phase grasse, au moins un filtre organique UV et au moins des nanopigments minéraux à base d'oxydes métalliques, dans le but d'augmenter le facteur de protection solaire.

Cette composition peut constituer notamment un stick et elle peut être utilisée en tant que produit de soin et/ou de maquillage du visage et/ou des lèvres

EP 1 530 964 A1

## Description

La présente demande concerne une composition solide photoprotectrice comprenant dans un milieu physiologiquement acceptable :

a) au moins une phase grasse,
b) au moins un filtre organique UV,
c) au moins des nanopigments minéraux à base d'oxydes métalliques,
d) au moins un sel d'amidon estérifié par l'anhydride octénylsuccinique.

La présente demande concerne également l'utilisation d'au moins un sel d'amidon estérifié par l'anhydride octénylsuccinique dans une composition solide photoprotectrice comprenant au moins une phase grasse, au moins un filtre organique UV et au moins des nanopigments minéraux à base d'oxydes métalliques, dans le but d'augmenter le facteur de protection solaire.

Il est bien connu que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection de la peau ont été proposées à ce jour.

Elles contiennent en général, à des concentrations diverses, un ou plusieurs filtres organiques classiques liposolubles et/ou des filtres organiques classiques hydrosolubles capables d'absorber sélectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV.

Pour obtenir des compositions solaires ayant des facteurs de protection solaire élevés afin de pouvoir les rendre plus efficaces et réduire les teneurs en filtres UV organiques pour des raisons de tolérance cutanée, il est fréquent d'utiliser des systèmes filtrants contenant des filtres UV organiques associés à des agents filtrant les radiation UV inorganiques tels que les nanopigments d'oxyde métallique ($TiO_2$ ou ZnO).

Les formes de formulation les plus utilisées pour les produits antisolaires sont les émulsions. On peut citer d'une part les émulsions directes de type huile-dans-eau (c'est à dire un support cosmétiquement et/ou dermatologiquement acceptable constitué d'une phase continue dispersante aqueuse et d'une phase discontinue dispersée grasse) bien les émulsions inverses du type eau-dans-huile (phase aqueuse dispersée dans une phase grasse continue), qui contient, à des concentrations diverses, un ou plusieurs filtres organiques classiques liposolubles et/ou des filtres organiques classiques hydrosolubles Dans de telles émulsions, les filtres hydrophiles sont présents dans la phase aqueuse et les filtres lipophiles sont présents dans la phase grasse. Dans de telles émulsions, les nanopigments d'oxydes métalliques selon leur caractère hydrophile ou hydrophobe sont dispersés dans la phase aqueuse ou huileuse.

Ces émulsions se présentent généralement sous forme de crèmes, de lotions ou de laits. Leur étalement et leur dépôt sont difficiles sur les lèvres ou sur certaines zones sensibles du visage comme le contour des yeux, le nez, les pommettes ou les cicatrices du fait que le produit peut couler sur la zone à protéger au moment de l'utilisation.

Pour résoudre ce problème, on connaît dans l'industrie cosmétique dans le domaine du soin des lèvres ou de ces zones sensibles du visage de nombreux produits se présentant sous forme solide. On peut notamment citer, dans le domaine du soin des lèvres, les crayons réparateurs des lèvres, les sticks solaires et les sticks hydratants. Il est en effet particulièrement intéressant de disposer de produits sous forme solide dans la mesure où de tels produits sont très pratiques utiliser et sont facilement transportables. Ils permettent un bon étalement et un bon dépôt sur les lèvres ou d'autres zones du visage.

Les sticks destinés à la photoprotection des lèvres et du visage actuels contiennent comme les émulsions solaires des systèmes filtrants contenant à la fois des filtres UV organiques et des filtres minéraux.

Il existe un besoin de plus en plus important d'obtenir dans ce type de support des facteurs de protection solaire élevés sans augmenter le taux de filtres UV présents dans la composition pour des raisons de tolérance cutanée.

La demanderesse a découvert de manière surprenante, que l'ajout d'au moins un sel d'amidon estérifié par l'anhydride octénylsuccinique dans une composition solide photoprotectrice à base de filtres organiques UV et de nanopigments d'oxydes métalliques permettait d'augmenter de façon substantielle le facteur de protection solaire (SPF) sans affecter les propriétés de stabilité et de délitement du stick. Cette augmentation sensible du SPF dans les sticks solaires à base de filtres UV organiques et de nanopigments est d'autant plus surprenante que ledit sel d'amidon estérifié par l'anhydride octénylsuccinique ne permet pas d'obtenir cet effet dans une émulsion solaire classique à base du même système filtrant.

Ainsi, la présente invention a pour objet une composition solide photoprotectrice comprenant dans un milieu physiologiquement acceptable :

a) au moins une phase grasse,
b) au moins un filtre organique UV,
c) au moins des nanopigments minéraux à base d'oxydes métalliques,
d) au moins un sel d'amidon estérifié par l'anhydride octénylsuccinique.

La présente invention a aussi pour objet l'utilisation d'au moins un sel d'amidon estérifié par l'anhydride octénylsuccinique dans une composition solide photoprotectrice comprenant au moins une phase grasse, au moins un filtre organique UV et au moins des nanopigments minéraux à base d'oxydes métalliques, dans le but d'augmenter le facteur de protection solaire.

La composition solide selon l'invention étant destinée à une application topique, notamment sur le visage et les lèvres, elle comprend un milieu physiologiquement acceptable. On entend ici par «milieu physiologiquement acceptable», un milieu non toxique et susceptible d'être appliqué sur le visage en particulier le nez, le contour des yeux, les joues ou bien sur les lèvres. La composition de l'invention peut constituer notamment une composition cosmétique ou dermatologique.

Par ailleurs, on entend par «composition solide» au sens de la présente invention, toute composition ne s'écoulant pas sous son propre poids, présentant une dureté telle que définie ci-dessous.

La dureté et donc la force de cisaillement de la composition selon l'invention doivent être de préférence telles que la composition soit autoportée, c'est-à-dire qu'elle se supporte elle-même en restant sous sa forme solide (par exemple bâton) et ne s'affaisse pas sous son poids comme le font les crèmes ou les liquides, et qu'elle puisse se déliter aisément pour former un dépôt satisfaisant sur la peau et les lèvres. La dureté des sticks obtenus est mesurée à 20°C au moyen d'un dynamomètre DFGHS 2 de la société INDELCO-CHATILLON se déplaçant à une vitesse de 100 mm/minute. Elle est exprimée comme la force de cisaillement (exprimée en grammes) nécessaire pour couper un stick de 12,7 mm de diamètre dans ces conditions. Dans la présente demande, la force de cisaillement de la composition va de préférence de 100 à 300 g, mieux de 120 à 250 g, et encore mieux de 150 à 220 g.

D'une manière générale, la composition selon l'invention constitue une composition solide et elle peut se présenter sous forme d'un produit coulé ou en coupelle ou sous forme d'un stick.

Les nanopigments minéraux à base d'oxydes métalliques utilisés dans la présente invention sont des poudres constituées de particules ayant une taille élémentaire moyenne inférieure à 100 nm et généralement comprise entre 5 nm et 100 nm, de préférence entre 10 et 50 nm. Les oxydes métalliques formant ces nanopigments peuvent être choisis notamment parmi les oxydes de titane, de fer, de zinc, de zirconium et de cérium, enrobés ou non. On préfère tout particulièrement parmi ces oxydes l'oxyde de titane, amorphe ou sous forme cristallisée (rutile et/ou anatase). Ces nanopigments peuvent être revêtus d'un enrobage hydrophobe. Ces nanopigments et leur utilisation en tant qu'agents photoprotecteurs sont connus et décrits par exemple dans les demandes EP 518772 et EP 518773.

Les nanopigments d'oxydes métalliques à enrobage hydrophobe utilisés selon l'invention peuvent par exemple avoir subi un ou plusieurs traitements avec un ou plusieurs composés choisis parmi l'alumine, la silice, les dérivés de l'aluminium (par exemple stéarate et laurate), les composés du silicium (par exemple silicones, polydiméthylsiloxanes, alcoxysilanes, siloxy-silicates), les composés du sodium, les oxydes de fer, les esters de fer (par exemple stéarate), les acides gras, les alcools gras et leurs dérivés (tels que l'acide stéarique, l'acide hydroxystéarique, l'alcool stéarylique, l'alcool hydroxystéarylique, l'acide laurique et leurs dérivés), la lécithine, les cires (par exemple la cire de carnauba), les polymères (méth)acryliques (par exemple les polyméthylmethacrylates), les composés fluorés (par exemple les composés perfluoroalkyle et les perfluoroalkyle éthers). Les oxydes peuvent aussi être traités par un mélange de ces composés ou ils peuvent comprendre plusieurs de ces enrobages successifs.

Plus particulièrement, les oxydes métalliques à enrobage hydrophobe utilisés dans la composition de l'invention peuvent être choisis parmi les nano-oxydes de titane traités par :

- l'alumine et l'acide stéarique, comme le produit commercialisé sous la dénomination UV-TITAN M160 par la société KEMIRA et le produit commercialisé sous la dénomination ST-430C par la société INANATA ;
- les polydiméthylsiloxanes (PDMS), comme les produits commercialisés sous la dénomination Eusolex T-2000 par

la société Merck, sous la dénomination UV TITAN X170 par la société ECKART, sous la dénomination Si-UFTR-Z par la société MYOSHI ;

- les alcoxysilanes, comme le produit commercialisé sous la dénomination COVASCREEN T1 par la société WACKHERR ;
- les composés perfluoroalkyle, comme le produit commercialisé sous la dénomination PF-7 TiO2 MT-600B par la société DAITO,
- les perfluoroalkyl éthers, comme le produit commercialisé sous la dénomination $TiO_2$ VF-25-3A par la société TOSHIKI ;
- les siloxy-silicates, par exemple comme le produit commercialisé sous la dénomination TSS-1 par la société ISK ;
- les polyméthylméthacrylates, comme par exemple le produit commercialisé sous la dénomination PW COVASIL S par la société WACKHERR ;
- la lécithine, comme le produit commercialisé sous la dénomination DUOTERC CW 5-25 par la société SACHTLEBEN ;
- la cire de carnauba, comme le produit commercialisé sous la dénomination UVT-PT 951101 par la société KEMIRA ;
- la silice et l'alumine, comme les produits commercialisés sous les dénominations MICROTITANIUM DIOXIDE MT 500 SA et MICROTITANIUM DIOXIDE MT 100 SA par la société TAYCA, et TIOVEIL FIN, TIOVEIL OP, TIOVEIL MOTG et TIOVEIL IPM par la société UNIQEMA ;
- l'alumine et le stéarate d'aluminium, comme le produit commercialisé sous la dénomination MICROTITANIUM DIOXIDE MT 100 T par la société TAYCA;
- l'alumine et le laurate d'aluminium, comme le produit commercialisé sous la dénomination MICROTITANIUM DIOXIDE MT 100 S par la société TAYCA;
- des oxydes de fer et le stéarate de fer, comme le produit commercialisé sous la dénomination MICROTITANIUM DIOXIDE MT 100 F par la société TAYCA;
- la silice, l'alumine et la silicone, comme les produits commercialisés sous les dénominations MICROTITANIUM DIOXIDE MT 100 SAS, MICROTITANIUM DIOXIDE MT 600 SAS et MICROTITANIUM DIOXIDE MT 500 SAS par la société TAYCA;
- l'octyltriméthoxysilane comme le produit commercialisé sous la dénomination T-805 par la société DEGUSSA ou le produit commercialisé sous la dénomination UVINUL $TiO_2$ par la société BASF ;
- l'alumine et la glycérine, comme le produit commercialisé sous la dénomination UVT-M212 par la société KEMIRA;
- l'alumine et la silicone, comme le produit commercialisé sous la dénomination UVT-M262 par de la société KEMIRA.

Ces nano-oxydes de titane enrobés hydrophobes peuvent se présenter sous forme de charge solide ou sous forme de dispersion dans un milieu huileux. Comme dispersions d'oxyde de titane enrobé, on peut citer par exemple les produits indiqués ci-dessus, commercialisés par la société UNIQEMA sous les dénominations TIOVEIL FIN (nano-oxyde de titane dispersé dans benzoate d'alcool $C_{12}$-$C_{15}$ avec, comme dispersant, un polycondensat d'acide hydrostéarique) et TIOVEIL MOTG (nano-oxyde de titane dispersé dans huile minérale/triglycérides avec, comme dispersant, un polycondensat d'acide hydroxystéarique); le produit commercialisé sous la dénomination COVASCREEN TI par la société WACKHERR (dispersion huileuse de $TiO_2$ enrobé par du triméthoxy-octylsilane à 60 %) ; le produit commercialisé sous la dénomination DAITOPERSION TI-30 par la société DAITO (dispersion de nano-oxyde de titane enrobé par du polyméthylhydrogènesiloxane dans cyclométhicone et diméthicone copolyol) ; le produit commercialisé sous la dénomination TIOSPERSE ULTRA par la société COLLABORATIVE LABORATORIES (nano-oxyde de titane enrobé par acide stéarique/alumine, dispersé dans du benzoate d'hydroxystéarate d'éthyl-2-hexyle); le produit commercialisé sous la dénomination MIBRID DISPERSION SAS4 5050 par la société MYOSHI (nano-oxyde de titane enrobé par alumine/acide stéarique puis par polydiméthylsiloxane, dispersé dans cyclopentasiloxane) ; le produit commercialisé sous la dénomination SPD-T1 par la société SHIN-ETSU (nano-oxyde de titane enrobé par un polymère acrylique greffé silicone et dispersé dans le cyclopentadiméthylsiloxane).

Comme oxyde de zinc utilisables dans la composition de l'invention, on peut citer par exemple les dispersions de nano-oxydes de zinc commercialisées sous les dénominations DAITOPERSION ZN-30 et Zn-50 par la société DAITO, qui sont des dispersions dans cyclopolyméthylsiloxane / polydiméthylsiloxane oxyéthyléné, contenant 30% ou 50% de nano-oxydes de zinc enrobés par le polyméthylhydrogènesiloxane.

On peut utiliser une ou plusieurs sortes d'oxydes métalliques enrobés dans la composition de l'invention.

On utilisera plus particulièrement des nanopigments de $TiO_2$ traités par l'octyltriméthoxysilane comme les produits commerciaux T-805 et UVINUL $TiO_2$.

Les nanopigments conformes à l'invention sont généralement présents dans les compositions selon l'invention dans des proportions allant de 0,01 à 20% en poids par rapport au poids total de la composition, et de préférence allant de 0,1 à 10% en poids par rapport au poids total de la composition.

Parmi les sels d'amidon estérifiés par l'anhydride octénylsuccinique utilisables selon l'invention, on peut citer les sels de calcium, sodium ou d'aluminium obtenus par réaction de l'anhydride octénylsuccinique avec un amidon de maïs et en particulier les sels d'amidon de maïs estérifiés suivants désignés ci-dessous sous leur nom INCI :

- Aluminium Starch Octenylsuccinate vendu notamment sous le nom commercial "DRY FLO PLUS" ou DRY FLO PURE par la société NATIONAL STARCH
- Sodium Starch Octenylsuccinate vendu notamment sous le nom commercial "CAPSUL" par la société NATIONAL STARCH ;
- Calcium Starch Octenylsuccinate vendu sous le nom commercial "SKIN FLOW-C" par la société MILDWEST GRAIN PRODUCTS.

On utilisera plus particulièrement l'Aluminium Starch Octenylsuccinate.

Les sels d'amidon estérifiés par l'anhydride octénylsuccinique conformes à l'invention sont de préférence présents dans des concentrations allant de 0,1 à 10 % en poids et plus préférentiellement de 1 à 5 % en poids par rapport au poids total de la composition.

Les filtres UV organiques conformes à l'invention sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US 5,237,071, US 5,166,355, GB2303549, DE 19726184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP1133981 et leurs mélanges.

Comme exemples de filtres organiques, on peut citer ceux désignés ci-dessous sous leur nom INCI :

<u>Dérivés de l'acide para-aminobenzoique :</u>

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

<u>Dérivés salicyliques :</u>

Homosalate vendu sous le nom « Eusolex HMS » par Rona/EM Industries,
Ethylhexyl Salicylate vendu sous le nom « NEO HELIOPAN OS » par HAARMANN et REIMER,
Dipropyleneglycol Salicylate vendu sous le nom « DIPSAL » par SCHER,
TEA Salicylate, vendu sous le nom « NEO HELIOPAN TS » par HAARMANN et REIMER,

<u>Dérivés du dibenzoylméthane :</u>

Butyl Methoxydibenzoylmethane vendu notamment sous le nom commercial « PARSOL 1789 » par ROCHE VITAMINS,
Isopropyl Dibenzoylmethane,

<u>Dérivés cinnamiques :</u>

Ethylhexyl Methoxycinnamate vendu notamment sous le nom commercial « PARSOL MCX » par ROCHE VITAMINS,
Isopropyl Methoxy cinnamate,
Isoamyl Methoxy cinnamate vendu sous le nom commercial « NEO HELIOPAN E 1000 » par HAARMANN et

REIMER,
Cinoxate, DEA Methoxycinnamate, Diisopropyl Methylcinnamate,
Glyceryl Ethylhexanoate Dimethoxycinnamate

Dérivés de β,β'-diphénylacrylate :

Octocrylene vendu notamment sous le nom commercial « UVINUL N539 » par BASF,
Etocrylene, vendu notamment sous le nom commercial « UVINUL N35 » par BASF,

Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial « UVINUL DS-49 » par BASF,
Benzophenone-12
2-(4-diethylamino-2-hydroxybenzoyl)-benzoate de n-hexyle vendu sous le nom commercial«UVINUL A PLUS » par BASF,

Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK ,
Benzylidene Camphor Sulfonic Acid fabriqué sous le nom «MEXORYL SL » par CHIMEX,
Camphor Benzalkonium Methosulfate fabriqué sous le nom « MEXORYL SO» par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial «NEO HELIOPAN AP » par HAARMANN et REIMER,

Dérivés de la triazine :

Anisotriazine vendu sous le nom commercial «TINOSORB S » par CIBA GEIGY,
Ethylhexyl triazone vendu notamment sous le nom commercial « UVINUL T150 » par BASF,
Diethylhexyl Butamido Triazone vendu sous le nom commercial « UVASORB HEB » par SIGMA 3V,

Dérivés du phenyl benzotriazole :

Drometrizole Trisiloxane vendu sous le nom « Silatrizole » par RHODIA CHIMIE Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial «TINOSORB M» par CIBA SPECIALTY CHEMICALS,

Dérivés anthraniliques :

Menthyl anthranilate vendu sous le nom commercial commercial «NEO HELIOPAN MA» par HAARMANN et REIMER,

Dérivés d'imidazolines :

Ethylhexyl Dimethoxybenzylidene Dioxoimidazoline Propionate,

Dérivés du benzalmalonate :

Polyorganosiloxanes à fonction benzalmalonate tels que le Polysilicone-15 vendu sous la dénomination commerciale « PARSOL SLX » par ROCHE VITAMINS.

Dérivés de 4,4-diarylbutadiène :

-1,1-dicarboxy (2,2'-diméthyl-propyl)-4,4-diphénylbutadiène

Dérivés de benzoxazole :

2,4-bis-[5-1 (diméthylpropyl)benzoxazol-2-yl-(4-phenyl)-imino]-6-(2-ethylhexyl)-imino-1,3,5-triazine vendu sous le nom Uvasorb K2A par Sigma 3V ;
et leurs mélanges.

Les filtres UV organiques préférentiels sont choisis parmi

Ethylhexyl Salicylate,
Ethylhexyl Methoxycinnamate
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
et leurs mélanges.

Les filtres UV organiques conformes à l'invention sont généralement présents dans les compositions selon l'invention dans des proportions allant de 0,01 à 20% en poids par rapport au poids total de la composition, et de préférence allant de 0,1 à 10% en poids par rapport au poids total de la composition.

Selon un mode particulier de réalisation de l'invention, la composition comprend un système filtrant contenant au moins le Butyl Methoxydibenzoylmethane, l'Octocrylene et des nanopigments d'oxyde de titane.

Selon un autre mode particulier de réalisation de l'invention, la composition comprend un système filtrant contenant au moins le Butyl Methoxydibenzoylmethane, l'Octocrylene, le Drometrizole Trisiloxane et des nanopigments d'oxyde de titane.

La composition de l'invention comprend de préférence une seule phase grasse, et cette phase grasse est de préférence une phase continue.

Les compositions de l'invention peuvent être des compositions anhydres, c'est-à-dire exemptes d'eau ou de composés hydrophiles, mais elles peuvent aussi ne pas être anhydres et comprendre alors jusqu'à 10 % en poids de phase hydrophile par rapport au poids total de la composition, de préférence 1 à 5 % en poids de phase de phase hydrophile par rapport au poids total de la composition, la phase hydrophile étant constituée d'eau seule, ou d'eau et d'additifs hydrophiles et hydrosolubles tels que polyols, gélifiants et/ou actifs. Si cette phase hydrophile est présente, elle est de préférence dispersée dans la phase grasse qui forme une phase continue.

Selon un mode particulier de réalisation de l'invention, la composition comprend une phase hydrophile comprenant au moins un filtre UV hydrosoluble.

Parmi les filtres UV hydrosolubles utilisables, on peut citer

Phenylbenzimidazole Sulfonic Acid,
Terephthalylidene Dicamphor Sulfonic Acid,
Disodium Phenyl Dibenzimidazole Tetra-sulfonate,
Benzophenone-4.

On utilisera plus particulièrement comme filtre UV hydrosoluble le Terephthalylidene Dicamphor Sulfonic Acid fabriqué sous le nom « MEXORYL SX » par CHIMEX,

Selon un mode particulier de réalisation de l'invention, la composition est anhydre, c'est-à-dire qu'elle ne comporte que la phase grasse, ou quasiment anhydre, c'est-à-dire qu'elle comporte moins de 5 % en poids de phase hydrophile (eau et/ou additifs hydrophiles ou hydrosolubles).

La phase grasse de la composition selon l'invention comprend en général au moins une huile et au moins une cire

Par "huile", au sens de la présente invention, on entend tout milieu non aqueux liquide à température ambiante (25°C) et pression atmosphérique (760 mm de Hg), physiologiquement acceptable.

Par "cire", au sens de la présente invention, on entend tout composé gras lipophile, solide à température ambiante (25°C), à changement d'état solide/liquide réversible, ayant une température de fusion supérieure à 40°C et pouvant aller jusqu'à 200°C, et présentant à l'état solide une organisation cristalline anisotrope.

Les huiles conformes à l'invention peuvent être des huiles hydrocarbonées et/ou siliconées et/ou fluorées. Elles peuvent être d'origine animale, végétale, minérale ou synthétique.

On entend par « huile hydrocarbonée », toute huile comportant majoritairement des atomes de carbone et d'hydrogène, et éventuellement des groupements ester, éther, fluoré, acide carboxylique et/ou alcool. En outre; les huiles utilisées peuvent être volatiles et/ou non volatiles.

Par "huile volatile", on entend une huile susceptible de s'évaporer à température ambiante d'un support sur lequel elle a été appliquée, autrement dit une huile ayant une tension de vapeur mesurable à 25°C et 1 atmosphère, par exemple supérieure à 0 Pa, en particulier allant de 10-3 à 300 mm de Hg (0,13 Pa à 40.000 Pa).

On peut notamment citer comme huiles volatiles, les huiles siliconées volatiles, telles que les silicones volatiles cycliques ou linéaires. On peut également citer les huiles volatiles hydrocarbonées telles que les isoparaffines, et les huiles fluorées volatiles.

Parmi les huiles pouvant être utilisées dans la composition de l'invention, certaines sont polaires et d'autres sont apolaires (c'est-à-dire non polaires).

Les huiles polaires comportent dans leur structure chimique au moins un groupement polaire non ionique, et de préférence au moins deux groupements polaires non ioniques ou ioniques tels que les groupements suivants :

- COOH;
- OH mono ou disubstitué (primaire ou secondaire) ;
- $PO_4$;
- NHR ; $NR_1R_2$, $R_1$ et $R_2$ formant éventuellement un cycle et représentant un radical alkyle ou alcoxy linéaire ou ramifié en $C_1$ à $C_{20}$, ou

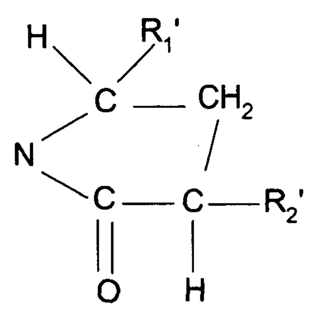

où $R_1$' et $R_2$' peuvent représenter l'hydrogène ou une chaîne alkyle ou alkoxy linéaire ou ramifiée en $C_1$ à $C_{20}$.

La polarité peut être décrite par le paramètre de solubilité de Hansen δa. En effet, ce paramètre caractérise,

pour un constituant donné, l'énergie correspondant aux interactions polaires (δp) et de types liaisons hydrogène (δh) existant entre les molécules de ce constituant.

$$\delta_a = \sqrt{\delta_p^2 + \delta_h^2}$$

Les huiles apolaires ont une valeur de δa égale à 0. En particulier, les huiles apolaires selon l'invention peuvent être en particulier choisies parmi :

- les huiles de silicone, telles que les polydiméthylsiloxanes (PDMS) volatils ou non, linéaires ou cycliques, liquides à température ambiante ; les silicones phénylées comme les phényltriméthicones, les phényldiméthicones, les phényltriméthylsiloxydiphénylsiloxanes, des diphényldiméthicones, les diphénylméthyldiphényltrisiloxanes, les 2-phényléthyl triméthylsiloxysilicates ;
- les hydrocarbures, linéaires ou ramifiés, d'origine synthétique ou minérale, comme les huiles de paraffine, volatiles ou non volatiles, et leurs dérivés ; l'huile de vaseline ; la lanoline liquide ; les polydécènes ; le polyisobutène hydrogéné tel que l'huile de PARLEAM ®; le squalane ; l'isoparaffine hydrogénée ; l'isohexadecane ; l'isododecane ;
- et leurs mélanges.

Les huiles polaires ont une valeur de δa différente de 0, c'est-à-dire supérieure à 0. En particulier, les huiles polaires utilisées dans la composition de l'invention peuvent être choisies parmi :

- les huiles d'origine végétale, huiles hydrocarbonées à forte teneur en triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées. Comme huiles d'origine végétale, on peut citer notamment les huiles de jojoba, de germes de blé, de maïs, de tournesol, de beurre de karité, de ricin, d'amandes douces, de macadamia, d'abricot, de soja, de coton, de luzerne, de pavot, de potimarron, de sésame, de courge, de colza, d'avocat, de noisette, de pépins de raisin ou de cassis, d'onagre, de millet, d'orge, de quinoa, d'olive, de seigle, de carthame, de bancoulier, de passiflore, de rosier muscat, de coriandre ; ou encore les triglycérides des acides caprylique/caprique comme ceux vendus par la société STEARINERIES DUBOIS ou ceux vendus sous les dénominations MIGLYOL 810, 812 et 818 par la société DYNAMIT NOBEL ;
- les huiles de synthèse ou esters de synthèse de formule $R_5COOR_6$ dans laquelle $R_5$ représente le reste d'un acide gras linéaire ou ramifié comportant de 1 à 40 atomes de carbone et $R_6$ représente une chaîne hydrocarbonée notamment ramifiée contenant de 1 à 40 atomes de carbone à condition que : $R_5 + R_6$ soit ≥ 10, comme par exemple l'huile de Purcellin (octanoate de cétostéaryle), l'isononanoate d'isononyle, le benzoate d'alcool en $C_{12}$ à $C_{15}$, le myristate d'isopropyle, le palmitate d'éthyl 2-hexyle, l'isostéarate d'isostéaryle, l'isostéarate d'isopropyle, des octanoates, décanoates ou ricinoléates d'alcools ou de polyalcools ; les esters hydroxylés comme le lactate d'isostéaryle, le malate de di-isostéaryle ; le $C_{12}$-$C_{15}$ alkylbenzoate ; et les esters du pentaérythritol ;
- les éthers de synthèse ayant de 10 à 40 atomes de carbone ;
- les alcools gras en $C_8$ à $C_{26}$, comme l'alcool oléique, l'alcool isostéarylique, et l'octyldodécanol ;
- leurs mélanges.

La quantité d'huile(s) peut aller par exemple de 20 à 80 % en poids et de préférence de 30 à 70 % en poids par rapport au poids total de la composition.

Les cires conformes à l'invention peuvent être choisies parmi les cires d'origine naturelle, notamment d'origines végétale ou animale, parmi les cires d'origine minérale, parmi les cires d'origine synthétique, et leurs mélanges. Comme cires pouvant être utilisées dans la composition de l'invention, on peut citer par exemple la cire d'abeilles, la cire de Montan, la cire de Carnauba, la cire de Candellila, la cire de Chine, la cire de lin, la cire de sapin, la cire de coton, la cire d'Ouricoury, la cire de lignite, la cire de son de riz, la cire de canne à sucre, la cire du Japon, la cire de fibres de liège, les cires de paraffine, les cires microcristallines, la cire de lanoline, les ozokérites, les huiles hydrogénées ayant une température de fusion supérieure à 40°C (environ), comme l'huile de jojoba hydrogénée, les cires de polyéthylène qui sont issues de la polymérisation de l'éthylène, les cires obtenues par synthèse de Fischer-Tropsch, les esters d'acides gras et les glycérides concrets (c'est-à-dire solides) à 40°C, les cires de silicone comme les alkyle, alcoxy et/ou esters de poly(di)méthylsiloxane solides à 40°C ; et leurs mélanges.

La quantité totale de cire(s) peut aller par exemple de 5 à 40 % en poids et de préférence de 10 à 30 % en poids par rapport au poids total de la composition.

La phase grasse peut comprendre en outre au moins un corps gras de consistance pâteuse de type beurre tel que le beurre de karité et le beurre de cacao.

Comme indiqué ci-dessus, la composition de l'invention peut comprendre de 0 à 10 % en poids d'une phase

hydrophile, par rapport au poids total de la composition, et mieux de 1 à 5 % en poids, pouvant comprendre de l'eau et/ou des additifs hydrophiles ou hydrosolubles (filtres hydrosolubles, vitamines et/ou gélifiants par exemple). Elle peut notamment comprendre des hydratants tels que la glycérine. Les constituants hydrophiles éventuellement présents sont de préférence dispersés dans la phase grasse constituée des huiles et cires.

Les compositions selon l'invention peuvent comprendre en plus des nanopigments d'oxyde métallique des particules choisies parmi les pigments, les nacres, les charges, et leurs mélanges.

Ces pigments, nacres, et charges additionnels sont choisis parmi ceux habituellement utilisés dans les compositions cosmétiques.

Par "pigments" , il faut comprendre des particules blanches ou colorées, minérales ou organiques, destinées à colorer et/ou opacifier la composition.

Par "charges", il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.

Par nacres, il faut comprendre des particules irisées qui réfléchissent la lumière.

Les pigments additionnels peuvent être blancs ou colorés, minéraux et/ou organiques, de taille micrométrique ou nanométrique. On peut citer par exemple, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.

Parmi les nacres utilisables dans la composition de l'invention, on peut citer par exemple le mica recouvert d'oxyde de titane, d'oxyde de fer, de pigment naturel ou d'oxychlorure de bismuth ainsi que le mica titane coloré.

Les charges peuvent être minérales ou de synthèse, lamellaires ou non lamellaires. Comme charges utilisables dans la composition de l'invention, on peut citer par exemple le talc, le mica, la silice, le kaolin, les poudres de Nylon, les poudres de polyéthylène, le Téflon, l'amidon modifié ou non, le micatitane, la nacre naturelle, le nitrure de bore, les microsphères telles que l'EXPANCEL (NOBEL INDUSTRIE), le POLYTRAP (DOW CORNING) et les microbilles de résine de silicone (TOSPEARLS de TOSHIBA, par exemple).

Les compositions selon l'invention peuvent comprendre en plus une matière colorante qui peut être choisie parmi les colorants lipophiles ou les colorants hydrophiles habituellement utilisés dans les compositions cosmétiques ou dermatologiques, ainsi que parmi les pigments et les nacres décrits ci-dessus; et leurs mélanges. Cette matière colorante est généralement présente à raison de 0,01 à 40 % en poids et de préférence de 5 à 25 % en poids par rapport au poids total de la composition.

La composition selon l'invention peut comprendre en outre tout additif usuellement utilisé dans le domaine considéré, notamment le domaine cosmétique, tel que les antioxydants ; les parfums ; les huiles essentielles ; les conservateurs ; les actifs cosmétiques ; les vitamines comme la vitamine E (tocophérol) et ses dérivés (par exemple acétate), la vitamine A (rétinol) et ses dérivés (par exemple palmitate de rétinyle), la vitamine C (acide ascorbique) et ses dérivés (par exemple palmitate d'ascorbyle), les dérivés de ces vitamines étant notamment des esters dont le palmitate et l'acétate; les acides gras essentiels ; les sphingolipides et céramides ; les tensioactifs ; les polymères ; et leurs mélanges. Ces additifs peuvent être présents dans la composition à raison de 0 à 20 % en poids par rapport au poids total de la composition.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Comme indiqué ci-dessus, la composition selon l'invention se présente sous forme solide. On entend par là qu'on n'observe aucun affaissement de la composition en dehors du récipient la comprenant, en l'absence de stimulation mécanique ou thermique (chauffage notamment).

Les procédés de fabrication des compositions selon l'invention ne différent en rien des procédés classiquement utilisés en cosmétique et parfaitement connus de l'homme de l'art.

Les compositions selon l'invention peuvent notamment constituer un produit de soin et/ou un produit de maquillage des lèvres et/ou du visage notamment le nez, le contour des yeux et les joues.

Les produits de maquillage sont le plus souvent colorés et contiennent généralement des pigments. Sous forme de produits de maquillage, les compositions de l'invention peuvent constituer avantageusement un fond de teint, un rouge à lèvres, un fard à joues, un fard à paupières.

Ce produit peut être sous forme d'une poudre coulée, d'un produit en coupelle (fond de teint, fard à joues, fard à paupières) ou d'un produit sous forme de bâton (rouge à lèvres ou stick de soin des lèvres). Selon un mode préféré de réalisation de l'invention, elle se présente sous forme de bâton (stick), plus particulièrement pour la photoprotection des lèvres et/ou du visage.

**Exemples de sticks solaires :**

| Ingrédients | Stick 1 | Stick 2 |
|---|---|---|
| Amidon de maïs estérifié par anhydride octenylsuccinique, sel d'aluminium (DRY FLO PLUS - NATIONAL STARCH) | 0 | 5 g |
| Cire de Carnauba | 5 g | 5 g |
| Cire de Candelilla | 8 g | 8g |
| Cire de polyéthylène | 7 g | 7 g |
| Silice pyrogénée hydrophobe (AEROSIL R 972 - DE GUSSA) | 0,23 g | 0,23 g |
| Huile de ricin | 12 g | 12 g |
| Isoparaffine (6-8 moles d'isobutylène) hydrogénée | 15 g | 15 g |
| Beurre de karité | 3,5 g | 3,5 g |
| Beurre de cacao | 3,5 g | 3,5 g |
| 4-tertiobutyl-4'-méthoxy-dibenzoylméthane (PARSOL 1789-ROCHE VITAMINS) | 3 g | 3 g |
| Octocrylene (UVINUL N539-BASF) | 10 g | 10 g |
| Drometrizole Trisiloxane (SILATRIZOLE - RHODIA CHIMIE) | 1 g | 1 g |
| Oxyde de Titane rutile (25-40 nm) traité octyl triméthoxysilane (UVINUL $TIO_2$ - BASF) | 1 g | 1 g |
| Terephthalylidene Dicamphor Sulfonic Acid (Mexoryl SX-CHIMEX) | 1,5 g | 1,5 g |
| Talc | 3 g | 3 g |
| Mélange de tocophérols naturels dans huile de soja (50/50) | 0,2 g | 0,2 g |
| Sorbitol cristallisé | 0,5 g | 0,5 g |
| Sel pentasodique de l'acide éthylène diamine tétraméthylène phosphonique (DEQUEST 2046 -SOLUTIA) | 0,15 g | 0,15 g |
| Ascorbyl glucoside (HAYASHIBARA) | 0,27 g | 0,27 g |
| Eau désionisée | 0,9 g | 0,9 g |
| Triéthanolamine | qs | qs |
| Parfum | qs | qs |
| Triglycérides acides caprylique/caprique (60/40) (MYGLYOL 812-DYNAMIT NOBEL) | qsp 100 g | qsp 100 g |

**Mode opératoire :**

On mélange l'eau, le Terephthalylidene Dicamphor Sulfonic Acid, l'ascorbyl glucoside, le sorbitol , le D-panthénol et le sel pentasodique de l'acide éthylène diamine tétraméthylène phosphonique (phase aqueuse).
On fait fondre à 85°C les cires, les huiles et les beurres, les tocophérols et le parfum. On ajoute ensuite les filtres solaires liposolubles, le talc, l'amidon modifié et le dioxyde de titane. On ajoute la silice dispersée dans l'huile de ricin. On ajoute la phase aqueuse. On homogénéise pendant 10 minutes et on coule dans des moules à sticks.

**Mesure du SPF in vivo**

Pour chacun des sticks solaires 1 et 2 ainsi préparés, on a ensuite déterminé le facteur de protection solaire (SPF) qui lui était attaché. La mesure du facteur de protection solaire a été effectuée sur les 5 volontaires selon la méthode COLIPA référence 94/289 Octobre 1984.
Le facteur de protection solaire (SPF) a été alors calculé mathématiquement par le rapport du temps d'irradiation qui a été nécessaire pour atteindre le seuil érythématogène avec le filtre UV (zone protégée) au temps qui a été nécessaire pour atteindre le seuil érythématogène sans filtre UV (zone non protégée). Les résultats en facteur de

protection solaire moyen (moyenne sur les 5 volontaires) obtenus sont rassemblés dans le tableau donné ci-dessous.

| Compositions | SPF moyen in vivo |
|---|---|
| **Stick N°1 (art antérieur)** | 22 ± 3,1 |
| **Stick N°2 (invention)** | 40 ± 12,2 |

L'ajout de l'Aluminium Starch Octenylsuccinate dans le stick solaire 2 permet d'augmenter de manière importante (ie : environ 2 fois plus) le SPF par rapport au stick solaire 1.

**Essais sur l'influence de l'Aluminium Starch Octenylsuccinate sur le SPF d'une formulation sous forme d'émulsion eau/huile liquide.**

On réalise les émulsions E/H suivantes :

| Ingrédients | Ex A | Ex B |
|---|---|---|
| Amidon de maïs estérifié par anhydride octenylsuccinique , sel d'aluminium (DRY FLO PLUS - NATIONAL STARCH) | 0 | 5 g |
| Alkylbenzoate en $C_{12}$-$C_{15}$ | 6g | 6g |
| Cyclohexasiloxane | 8g | 8g |
| Cyclopentasiloxane | 8g | 8g |
| Mélange de tocophérols naturels dans huile de soja (50/50) | 0,2 g | 0,2 g |
| Laury PEG/PPG 18/18 Méthicone (DC 5200-DOW CORNING) | 2g | 2g |
| PEG-30 Dipolyhydroxystéarate (ARLACEL P135- UNIQUEMA) | 2g | 2g |
| 4-tertiobutyl-4'-méthoxy-dibenzoylméthane (PARSOL 1789-ROCHE VITAMINS) | 3 g | 3 g |
| Octocrylene (UVINUL N539-BASF) | 10 g | 10 g |
| Drometrizole Trisiloxane (SILATRIZOLE - RHODIA CHIMIE) | 1g | 1g |
| Oxyde de Titane rutile (25-40 nm) traité octyl triméthoxysilane (UVINUL $TiO_2$ - BASF) | 1 g | 1 g |
| Terephthalylidene Dicamphor Sulfonic Acid (Mexoryl SX-CHIMEX) | 1,5 g | 1,5 g |
| Propyleneglycol | 4 g | 4g |
| Glycérine | 4g | 4g |
| Ethylhexylglycérine (SENSIVA SC50- SCHULKE & MAYR) | 0,75 | 0,75 |
| Alcool dénaturé | 4,20 g | 4,20g |
| Sel pentasodique de l'acide éthylène diamine tétraméthylène phosphonique (DEQUEST 2046 -SOLUTIA) | 0,1g | 0,1g |
| Nacl | 1g | 1g |
| Triéthanolamine | qs | qs |
| Eau désionisée | qsp 100 g | qsp 100g |

Chaque émulsion A et B est appliquée sur des plaques de Quarz + Transpore à raison de 1,4 $mg/cm^2$.

Pour chaque formulation A et B, le Facteur de Protection Solaire (SPF) a été mesuré selon la méthode in vitro tel que décrite dans l'article de DIFFEY et al. dans J. Soc. Cosmet. Chem. 40-127-133 (1989) ; cette méthode consiste à determiner le SPF monochromatique tous les 5 nm dans le domaine de longueurs d'onde 290-400 nm et de calculer le SPF selon une équation mathématique.

Les mesures sont effectuées sur 4 zones de la plaque et chaque formulation est testée 5 fois (5 plaques). Les SPF moyens sont indiqués dans le tableau suivant :

| Compositions | SPF moyen in vitro |
|---|---|
| **Emulsion A** | 33,0 ± 4,2 |
| **Emulsion B** | 26,9 ± 2,4 |

L'ajout de l'Aluminium Starch Octenylsuccinate dans l'émulsion B n'augmente pas le SPF par rapport à l'émulsion A.

Il n'a donc aucun effet sur le SPF d'une composition solaire du type émulsion E/H contenant un système filtrant constitué de 1% de nanopigments de $TiO_2$ et des filtres UV organiques : 3% de 4-tertiobutyl-4'-méthoxy-dibenzoylméthane, 10% d'Octocrylene, 1% de Drometrizole Trisiloxane et 1,5% de Terephthalylidene Dicamphor Sulfonic Acid contrairement à un stick solaire selon l'invention contenant le même système filtrant.

## Revendications

**1.** Composition solide photoprotectrice **caractérisée par le fait qu'**elle comprend dans un milieu physiologiquement acceptable :

a) au moins une phase grasse,
b) au moins un filtre organique UV,
c) au moins des nanopigments minéraux à base d'oxydes métalliques,
d) au moins un sel d'amidon estérifié par l'anhydride octénylsuccinique.

**2.** Composition selon la revendication 1, **caractérisée par le fait qu'**elle présente une force de cisaillement de la composition allant de 100 à 300 g, mieux de 120 à 250 g, et encore mieux de 150 à 220 g.

**3.** Composition selon la revendication 1 ou 2, où les nanopigments minéraux à base d'oxydes métalliques ont une taille élémentaire moyenne comprise entre 5 nm et 100 nm, de préférence entre 10 et 50 nm.

**4.** Composition selon la revendication 3, où les nanopigments sont choisis parmi les oxydes de titane, de fer, de zinc, de zirconium et de cérium, enrobés ou non.

**5.** Composition selon la revendication 4, où les nanopigments sont choisis parmi les oxydes de titane sous forme amorphe ou sous forme cristallisée rutile et/ou anatase, enrobés ou non.

**6.** Composition selon l'une quelconque des revendications 1 à 5, où les nanopigments d'oxyde métallique sont à enrobage hydrophobe.

**7.** Composition selon la revendication 6, où les nanopigments à enrobage hydrophobe, subissent un ou plusieurs traitements avec un ou plusieurs composés choisis parmi l'alumine, la silice, les dérivés de l'aluminium, les composés du silicium, les composés du sodium, les oxydes de fer, les esters de fer, les acides gras, les alcools gras et leurs dérivés, la lécithine, les cires, les polymères (méth)acryliques, les composés fluorés ou leurs mélanges.

**8.** Composition selon l'une quelconque des revendications 1 à 7, où les nanopigments d'oxyde métallique sont choisis parmi les nanopigments d'oxide de titane enrobés par l'octyltriméthoxysilane.

**9.** Composition selon l'une quelconque des revendications 1 à 8, où les nanopigments d'oxyde métallique sont présents dans des proportions allant de 0,01 à 20% en poids par rapport au poids total de la composition, et de préférence allant de 0,1 à 10% en poids par rapport au poids total de la composition.

**10.** Composition selon l'une quelconque des revendications 1 à 9, où le sel d'amidon estérifié est choisi parmi les sels de calcium, sodium ou d'aluminium obtenus par réaction de l'anhydride octénylsuccinique avec un amidon de maïs.

**11.** Composition selon la revendication 10, où le sel d'amidon estérifié est choisi parmi l

- Aluminium Starch Octenylsuccinate

- Sodium Starch Octenylsuccinate
- Calcium Starch Octenylsuccinate

**12.** Composition selon la revendication 11, où le sel d'amidon estérifié est l'Aluminium Starch Octenylsuccinate.

**13.** Composition selon l'une quelconque des revendications 1 à 12, où le sel d'amidon estérifié par l'anhydride octénylsuccinique est présent dans des concentrations allant de 0,1 à 10% en poids et plus préférentiellement de 1 à 5% en poids par rapport au poids total de la composition.

**14.** Composition selon l'une quelconque des revendications 1 à 13, où le filtre UV organique est choisi parmi les anthranilates ; les dérivés cinnamiques ; les dérivés de dibenzoylméthane ; les dérivés salicyliques, les dérivés du camphre ; les dérivés de triazine ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres ; les dimères dérivés d'α-alkylstyrène ; les 4,4-diarylbutadiènes et leurs mélanges.

**15.** Composition selon la revendication 14, où le filtre UV organique est choisi parmi

Ethylhexyl Salicylate,
Ethylhexyl Methoxycinnamate
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
et leurs mélanges.

**16.** Composition selon l'une quelconque des revendications 1 à 15, où le filtre UV organique est présent dans des proportions allant de 0,01 à 20% en poids par rapport au poids total de la composition, et de préférence allant de 0,1 à 10% en poids par rapport au poids total de la composition.

**17.** Composition selon l'une quelconque des revendications 1 à 16, dans laquelle le système filtrant contient au moins le Butyl Methoxydibenzoylmethane, l'Octocrylene et des nanopigments d'oxyde de titane.

**18.** Composition selon l'une quelconque des revendications 1 à 16, dans laquelle le système filtrant contient au moins le Butyl Methoxydibenzoylmethane, l'Octocrylene, le Drometrizole Trisiloxane et des nanopigments d'oxyde de titane.

**19.** Composition selon l'une quelconque des revendications 1 à 18, comprenant une seule phase grasse et de préférence une phase grasse continue.

**20.** Composition selon l'une quelconque des revendications 1 à 19, **caractérisée par le fait qu'**elle anhydre.

**21.** Composition selon l'une quelconque des revendications 1 à 20, **caractérisée par le fait qu'**elle comprend jusqu'à

10 % en poids de phase hydrophile par rapport au poids total de la composition, de préférence de 1 à 5 % en poids de phase hydrophile par rapport au poids total de la composition.

**22.** Composition selon la revendication 21, **caractérisée par le fait qu'**elle comprend une phase hydrophile contenant au moins un filtre UV hydrosoluble.

**23.** Composition selon la revendication 21, **caractérisée par le fait que** le filtre UV hydrosoluble est le Terephthalylidene Dicamphor Sulfonic Acid,

**24.** Composition selon l'une quelconque des revendications 1 à 21, **caractérisée par le fait que** la phase grasse comprend au moins une huile et au moins une cire.

**25.** Composition selon la revendication 24, selon laquelle l'huile est choisie parmi les huiles hydrocarbonées et/ou siliconées et/ou fluorées, volatiles ou non volatiles, polaires ou apolaires, d'origine animale, végétale, minérale ou synthétique.

**26.** Composition selon la revendication 25, **caractérisée en ce que** la quantité d'huile va de 20 à 80 % en poids, et de préférence de 30 à 70 % en poids par rapport au poids total de la composition.

**27.** Composition selon la revendication 26, **caractérisée en ce que** la cire est choisie parmi les cires d'origine naturelle ; les cires d'origine minérale, les cires d'origine synthétique et leurs mélanges.

**28.** Composition selon l'une quelconque des revendications 24 à 27, **caractérisée en ce que** la quantité de cire va de 5 à 40 % en poids et de préférence de 10 à 30 % en poids par rapport au poids total de la composition.

**29.** Composition selon l'une quelconque des revendications 24 à 28, **caractérisée en ce que** la phase grasse comprend en plus au moins un corps gras de consistance pâteuse de type beurre.

**30.** Composition selon l'une quelconque des revendications 1 à 29, **caractérisée en ce qu'**elle contient en plus des particules choisies parmi les pigments, les nacres, les charges et leurs mélanges.

**31.** Composition selon l'une quelconque des revendications 1 à 30, **caractérisée en ce qu'**elle comprend au moins une matière colorante.

**32.** Composition selon l'une quelconque des revendications 1 à 31, **caractérisée en ce qu'**elle se présente sous forme de poudre coulée, d'un produit en coupelle ou sous forme de bâton (stick).

**33.** Utilisation d'une composition telle que définie dans l'une quelconque des revendications précédentes comme produit de maquillage des lèvres et/ou du visage.

**34.** Utilisation d'une composition telle que définie dans l'une quelconque des revendications précédentes comme produit de soin des lèvres et/ou du visage.

**35.** Utilisation d'une composition telle que définie dans l'une quelconque des revendications précédentes comme produit de photoprotection des lèvres et/ou du visage.

**36.** Utilisation d'au moins un sel d'amidon estérifié par l'anhydride octénylsuccinique tel que défini dans les revendications précédentes dans une composition solide photoprotectrice comprenant au moins une phase grasse, au moins un filtre organique UV et au moins des nanopigments minéraux à base d'oxydes métalliques, dans le but d'augmenter le facteur de protection solaire.

Office européen des brevets

# RAPPORT DE RECHERCHE EUROPEENNE

Numéro de la demande
EP 04 29 2444

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| D,X | EP 0 518 772 A (OREAL) 16 décembre 1992 (1992-12-16)<br>* exemple 10 *<br>* revendications 1-3,5,6 * | 1,3,4,6, 7,9-16, 24-27, 30-35 | A61K7/42 |
| Y | EP 0 418 443 A (NEUTROGENA CORP) 27 mars 1991 (1991-03-27)<br>* page 3, ligne 46 - ligne 53 *<br>* page 4, ligne 8 *<br>* page 4, ligne 15 - ligne 18 *<br>* page 4, ligne 39 - page 5, ligne 15 *<br>* page 6; exemple 3 *<br>* revendications 1,2,4,5,10 * | 1-36 | |
| Y | US 5 616 331 A (ALLARD ET AL) 1 avril 1997 (1997-04-01)<br>* colonne 1, ligne 44 - ligne 54 * | 1-36 | |
| A | EP 0 583 756 A (NAT STARCH CHEM INVEST) 23 février 1994 (1994-02-23)<br>* page 5; exemple 3 *<br>* revendications * | 1-36 | |

DOMAINES TECHNIQUES RECHERCHES (Int.Cl.7)

A61K

Le présent rapport a été établi pour toutes les revendications

| Lieu de la recherche | Date d'achèvement de la recherche | Examinateur |
|---|---|---|
| Berlin | 31 janvier 2005 | Pelli Wablat, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

3
EPO FORM 1503 03.82 (P04C02)

**ANNEXE AU RAPPORT DE RECHERCHE EUROPEENNE RELATIF A LA DEMANDE DE BREVET EUROPEEN NO.** EP 04 29 2444

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

31-01-2005

| Document brevet cité au rapport de recherche | | Date de publication | Membre(s) de la famille de brevet(s) | | | Date de publication |
|---|---|---|---|---|---|---|
| EP 0518772 | A | 16-12-1992 | FR | 2677543 | A1 | 18-12-1992 |
| | | | AT | 113466 | T | 15-11-1994 |
| | | | AU | 662149 | B2 | 24-08-1995 |
| | | | AU | 1820992 | A | 17-12-1992 |
| | | | CA | 2071277 | A1 | 14-12-1992 |
| | | | DE | 69200614 | D1 | 08-12-1994 |
| | | | DE | 69200614 | T2 | 16-03-1995 |
| | | | DK | 518772 | T3 | 18-04-1995 |
| | | | EP | 0518772 | A1 | 16-12-1992 |
| | | | ES | 2062874 | T3 | 16-12-1994 |
| | | | GR | 3014899 | T3 | 31-05-1995 |
| | | | JP | 5170635 | A | 09-07-1993 |
| | | | US | 5643557 | A | 01-07-1997 |
| | | | US | 5690915 | A | 25-11-1997 |
| | | | US | 5690917 | A | 25-11-1997 |
| | | | US | 5788955 | A | 04-08-1998 |
| | | | US | 5795565 | A | 18-08-1998 |
| EP 0418443 | A | 27-03-1991 | CA | 1330309 | C | 21-06-1994 |
| | | | JP | 3112921 | A | 14-05-1991 |
| | | | US | 4894222 | A | 16-01-1990 |
| | | | AU | 625368 | B2 | 09-07-1992 |
| | | | AU | 4152189 | A | 20-06-1991 |
| | | | DE | 68914455 | D1 | 11-05-1994 |
| | | | DE | 68914455 | T2 | 25-08-1994 |
| | | | EP | 0418443 | A1 | 27-03-1991 |
| | | | ES | 2055086 | T3 | 16-08-1994 |
| US 5616331 | A | 01-04-1997 | FR | 2715843 | A1 | 11-08-1995 |
| | | | BR | 9500555 | A | 26-09-1995 |
| | | | CA | 2142099 | A1 | 10-08-1995 |
| | | | CN | 1116519 | A ,C | 14-02-1996 |
| | | | DE | 69503933 | D1 | 17-09-1998 |
| | | | DE | 69503933 | T2 | 10-12-1998 |
| | | | EP | 0667144 | A1 | 16-08-1995 |
| | | | ES | 2122462 | T3 | 16-12-1998 |
| | | | JP | 2999132 | B2 | 17-01-2000 |
| | | | JP | 7252123 | A | 03-10-1995 |
| | | | KR | 151635 | B1 | 15-10-1998 |
| | | | US | 5756110 | A | 26-05-1998 |
| | | | US | 5730993 | A | 24-03-1998 |
| EP 0583756 | A | 23-02-1994 | US | 5256404 | A | 26-10-1993 |
| | | | CA | 2104080 | A1 | 15-02-1994 |
| | | | DE | 69306060 | D1 | 02-01-1997 |
| | | | DE | 69306060 | T2 | 13-03-1997 |

EPO FORM P0460

Pour tout renseignement concernant cette annexe : voir Journal Officiel de l'Office européen des brevets, No.12/82

## ANNEXE AU RAPPORT DE RECHERCHE EUROPEENNE RELATIF A LA DEMANDE DE BREVET EUROPEEN NO.

EP 04 29 2444

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

31-01-2005

| Document brevet cité au rapport de recherche | Date de publication | Membre(s) de la famille de brevet(s) | Date de publication |
|---|---|---|---|
| EP 0583756 A | | EP 0583756 A2 | 23-02-1994 |
| | | JP 2088524 C | 02-09-1996 |
| | | JP 6183944 A | 05-07-1994 |
| | | JP 8002776 B | 17-01-1996 |

EPO FORM P0460

Pour tout renseignement concernant cette annexe : voir Journal Officiel de l'Office européen des brevets, No.12/82